Europäisches Patentamt

(19) European Patent Office

Office européen des brevets

(11) Veröffentlichungsnummer: **0 228 614**
**B1**

(12) **EUROPÄISCHE PATENTSCHRIFT**

(45) Veröffentlichungstag der Patentschrifft:
**28.02.90**

(51) Int. Cl. ⁵: **A 61 B 6/14**

(21) Anmeldenummer: **86117089.2**

(22) Anmeldetag: **08.12.86**

(54) Zahnärztliche Röntgendiagnostikeinrichtung.

(30) Priorität: **20.12.85 DE 3545487**

(43) Veröffentlichungstag der Anmeldung:
**15.07.87 Patentblatt 87/29**

(45) Bekanntmachung des Hinweises auf die Patenterteilung:
**28.02.90 Patentblatt 90/09**

(84) Bennante Vertragsstaaten:
**DE FR GB IT**

(56) Entgegenhaltungen:
**DE-A-3 304 061**
**DE-A-3 434 369**
**US-A-4 228 356**

(73) Patentinhaber: **Siemens Aktiengesellschaft**
**Wittelsbacherplatz 2**
**D-8000 München 2 (DE)**

(72) Erfinder: **Döbert, Michael**
**Zedernstrasse 3**
**D-6143 Lorsch (DE)**

EP 0 228 614 B1

## Beschreibung

Die Erfindung bezieht sich auf eine zahnärztliche Röntgendiagnostikeinrichtung für Panorama-Schichtaufnahmen mit einer Röntgenstrahlenquelle und einem Halter für eine Filmkassette, welche beide, an einem gemeinsamen Träger gehaltert, einander gegenüberstehend angeordnet sind und mittels einer ersten Verstellvorrichtung um das Objekt bewegt werden, und mit einer Filmkassette, die mittels einer zweiten Verstellvorrichtung während der Aufnahme relativ zur Strahlenquelle bewegt wird wobei die Verstellvorrichtung für die Filmkassette einen im Gehäuse des Filmkassettenhalters in Strahlengsrichtung gesehen hinter der Schlitzblende angeordneten elektromotorischen Antrieb vorsieht, der mindestens ein Antriebsglied und wenigstens ein korrespondierend angeordnetes Gegenglied enthält, sowie mit einer im Strahlengang zwischen Strahlenquelle und Film angeordneten Schlitzblende. Eine zahnärztliche Röntgendiagnostikeinrichtung dieser Art ist aus DE-A-3 304 061 bekannt.

Bei zahnärztlichen Röntgendiagnostikeinrichtungen der vorgenannten Gattung ist es bekannt (Prospekt M-D 80/1361 von Ortopantomograph 10), den Film in eine etwa halbkreisförmig gestaltete Metallkassette einzulegen und diese starre Kassette dann in eine mit dem Träger verbundene Halterung einzulegen. Die Filmkassette wird bei der Aufnahme dann über geeignete mechanische Steuermittel, z. B. über Kurvenscheiben od. dgl., relativ zur Strahlenquelle verstellt.

Aus der DE-OS-3 005 203 ist es weiterhin bekannt, anstelle einer gebogenen eine geradlinige, jedoch ebenfalls starre Filmkassette vorzusehen und diese zusammen mit einem Filmkassettenhalter mittels eines gesonderten Antriebes entsprechend der Bewegung der Strahlenquelle um das Objekt zu verstellen. Hierzu ist in dem kastenförmig ausgebildeten Filmkassettenhalter, entsprechend der Bewegungsrichtung der Kassette, eine Gleitschiene angeordnet, auf der sich eine von einem Motor angetriebene Rolle abwälzen kann. Innerhalb des Filmkassettenhalters sitzt ein streifenförmiges weiteres Gleitstück, das sich im wesentlichen parallel zur Gleitschiene erstreckt. Der Motor wird so angesteuert, daß Halter und Filmkassette beim Drehen des Schwenkarmes synchron zur Bewegung der Röntgenstrahlenquelle angetrieben werden, d.h. Halter und Filmkassette werden kontinuierlich in Längsrichtung der Gleitschiene verschoben.

Starre Filmkassetten sind einerseits konstruktiv aufwendig - und so mit erhöhten Kosten verbunden - andererseits in der Handhabung nachteilig, insbesondere, weil der zu belichtende Film vor jeder Aufnahme in einer Dunkelkammer in die Kassette eingelegt werden muß. Eine solche Handhabung ist, wenn mehrere Aufnahmen hintereinander zu erstellen sind, besonders umständlich und zeitraubend. Eine Bevorratung mit mehreren vorbereiteten Kassetten scheidet häufig aus Platz- und auch Kostengründen aus. Größe und darüber hinaus auch Gewicht sind deshalb weitere Nachteile solcher starren Filmkassetten. Das Gewicht der Filmkassette und des Filmkassettenhalters wirkt sich weiterhin auch nachteilig auf die Konstruktion des gesamten Antriebes aus; die Baugröße auf die Handhabung. So ist die Filmkassette dem Bedienungspersonal beim Positionieren des Patientenkopfes zur Vorbereitung der Aufnahme störend im Wege (Blickfeld).

Der im Anspruch 1 angegebenen Erfindung liegt die Aufgabe zugrunde, eine Röntgendiagnostikeinrichtung der eingangs genannten Gattung anzugeben, mit der sich die angeführten Nachteile vermeiden lassen, mit der sich insbesondere ein einfacherer, kostengünstigerer Aufbau der gesamten Filmhalterung erzielen und die Handhabung verbessern läßt.

Die Verwendung von flexiblen, den zu belichtenden Film bereits beinhaltenden Filmkassetten, welche bisher nur bei Geräten für intraorale Aufnahmen eingesetzt worden sind, bei denen die Filmkassette außen am Patientenmund angelegt und die Strahlenquelle in den Patientenmund eingeführt wird, eine Durchstrahlung also von innen nach außen erfolgt, nun auch für Panorama-Schichtaufnahmen, erlaubt es, in Verbindung mit der für die Filmkassette vorgesehene Transporteinrichtung die gesamte Röntgendiagnostikeinrichtung wesentlich einfacher und leichter zu gestalten.

Der Transport der Filmkassette erfolgt vorteilhafterweise mit Hilfe von an der Kassette anliegenden und diese durch Friktion bewegenden Antriebsgliedern und entsprechenden Gegengliedern. Diese sind vorzugsweise Rollen und/oder Walzen, die in Dreiecksanordnung mit engem Abstand unmittelbar benachbart der Sekundärblende angeordnet sind und die Filmkassette so führen, daß sich Lufteinschlüsse zwischen Film und den an sich heute stets vorhandenen Verstärkerfolien vermeiden lassen. Solche Lufteinschlüsse, die zu Verwischungen auf dem Röntgenbild führen, sind an sich bei Verwendung flexibler Kassetten mit Verstärkerfolien nicht immer auszuschließen, weil dort, im Gegensatz zu den Metallkassetten, bei denen eine Verspannung von Film und Folie über die (starre) Kassette erfolgt, eine solche Verspannung an sich nicht vorhanden ist. Mit der erfindungsgemäß vorgeschlagenen Transporteinrichtung werden jedoch Verstärkerfolie und Film unmittelbar benachbart der Sekundärblende zusammen verspannt und so von Lufteinschlüssen befreit.

Der gesamte Antrieb ist vorteilhafterweise an einem Träger befestigt, der im Kassettenhalterungsgehäuse als komplette Baueinheit herausnehmbar gehalten ist. Zur Verbesserung der Servicefreundlichkeit besteht das Kassettenhalterungsgehäuse vorteilhafterweise aus zwei durch Längsteilung gebildeten Hälften, von denen die eine den kompletten Antrieb und die andere im wesentlichen nur die Sekundärblende enthält oder bildet.

Ein weiterer Vorteil läßt sich erzielen, wenn man, wie gemäß einer weiteren vorteilhaften Weiterbildung der Erfindung vorgeschlagen wird, den Filmkassettenhalter um eine außermittig, vorteilhafterweise am einen Ende des Halters angeordnete, vertikale Achslagerung schwenkbar anordnet. Wegen der sich mit der Schwenkbarkeit des Filmkassettenhalters ergebenden besseren Sicht, die die Helferin hat, wird das Einstellen des Patientenkopfes auf die richtige Lage, entsprechend der vorgesehenen Schichtaufnahme, erleichtert. Das Wegschwenken hat auch noch den weiteren wesentlichen Vorzug, daß mit einem Gerät sowohl normale Panoramaschichtaufnahmen als auch Fernaufnahmen vom Schädel, sogenannte Ceph-Aufnahmen, erstellt werden können, ohne daß hierzu konstruktiv aufwendige Maßnahmen, unter anderem Schwenken des Röntgenstrahlers oder sonstige größere Umbauten, notwendig sind.

Ein Ausführungsbeispiel der Erfindung wird nachfolgend anhand der Zeichnung näher erläutert.

Es zeigen:

Figur 1 eine Röntgendiagnostikeinrichtung der erfindungsgemäßen Gattung in schaubildlicher Darstellung,

Figur 2 eine Draufsicht auf das Gerät gemäß Figur 1 mit Darstellung des Verstellantriebes für den Träger von Strahlenquelle und Filmkassettenhalter,

Figuren 3 und 4 die eine Gehäusehälfte des Filmkassettenalters im Grund- und Aufriß,

Figur 5 die andere Gehäusehälfte im Grundriß, teilweise im Schnitt,

Figur 6 den Träger für den Antrieb der Filmkassette in Draufsicht,

Figur 7 den Träger in Seitenansicht, teilweise im Schnitt.

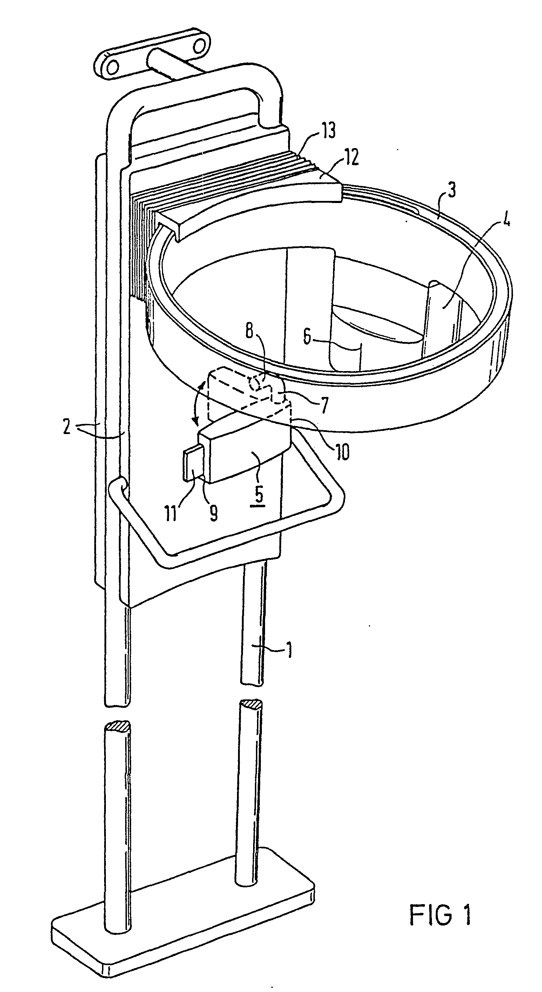

Die Figur 1 zeigt in einer schaubildlichen Darstellung ein zahnärztliches Röntgendiagnostikgerät zur Erstellung von Panorama-Schichtaufnahmen vom Kiefer eines Patienten, enthaltend einen aus zwei Rohren gebildeten Ständer 1, an dem ein Laufwagen 2 höhenverstellbar gehalten ist. Der Laufwagen 2 trägt einen Drehring 3, an dem einerseits eine Röntgenstrahlenquelle 4 und andererseits (diametral gegenüberliegend) ein Filmkassettenhalter 5 angeordnet ist. Während die Strahlenquelle 4, deren Strahlenaustrittsöffnung mit 6 bezeichnet ist, am Drehring 3 fest angeordnet ist, ist der Filmkassettenhalter 5 mittels vertikaler Achslagerung 7 an einem abgewinkelten Tragarm 8 in Richtung deseingezeichneten Pfeiles schwenkbar gehalten. Der Filmkassettenhalter kann so aus der gestrichelt eingezeichneten, für Schichtaufnahmen geeigneten Gebrauchsstellung in die mit durchgehenden Linien gezeichnete Schwenkstellung gebracht werden, die einerseits der Helferin das Positionieren des Patientenkopfes erleichtert und andererseits das Erstellen von Fernaufnahmen, sogenannten Ceph-Aufnahmen, ermöglicht, für welche bei bisherigen Geräteausführungen die Röntgenstrahlenquelle gedreht werden mußte.

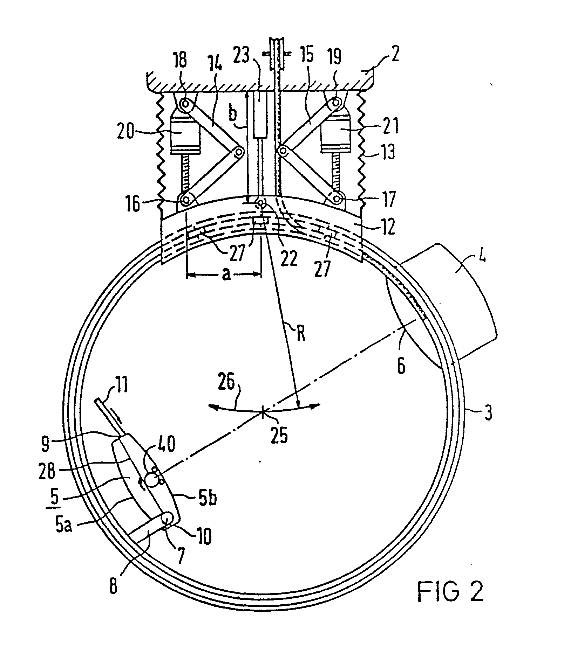

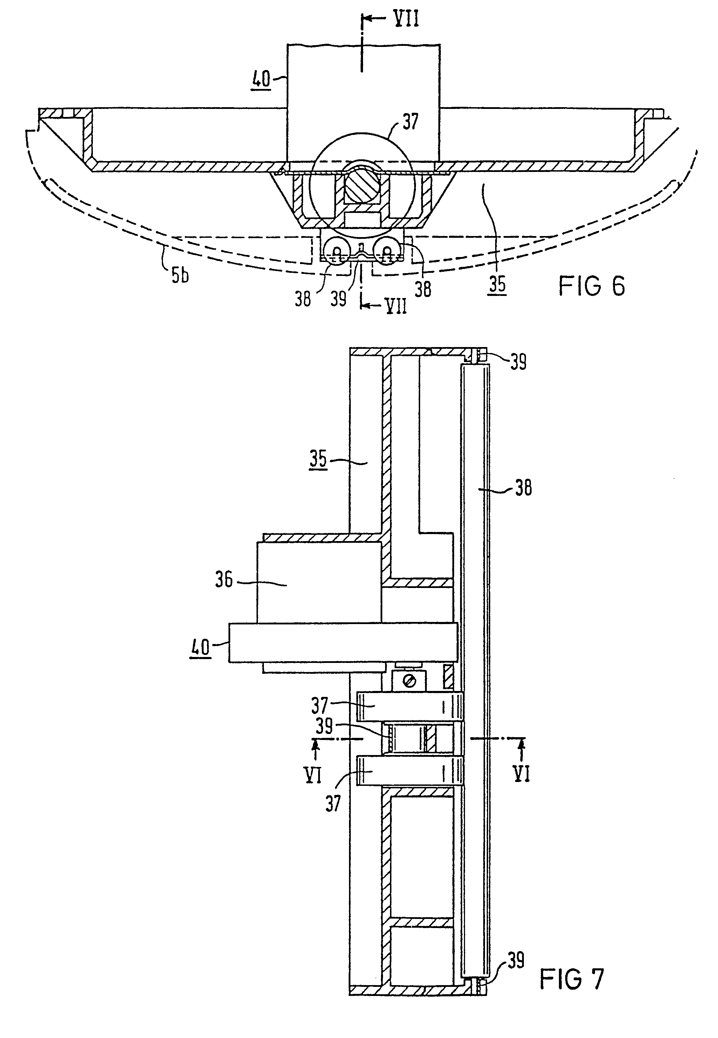

Der Filmkassettenhalter 5 enthält an beiden Stirnseiten schlitzförmige Ein- und Austrittsöffnungen 9, 10, über die die mit 11 bezeichnete Filmkassette eingeführt bzw. nach erstellter Aufnahme entnommen werden kann. Bei der Filmkassette 11 handelt es sich um eine flexible, mit Verstärkerfolie versehene Filmkassette, wie sie im Prinzip für die eingangs erwähnten intraoralen Aufnahmen verwendet werden. Der Transport der Filmkassette erfolgt mittels eines in Figur 2 allgemein mit 40 bezeichneten Antriebes, der anhand der Figuren 6 und 7 noch näher erläutert wird.

Bevor der Aufbau des Filmkassettenhalters 5 näher beschrieben wird, sei der verstellantrieb für den die Strahlenquelle 4 und den Filmkassettenhalter 5 tragenden Drehring 3 kurz erläutert.

Der Drehring 3 ist ein in sich geschlossener Ring, der in einem Lagerteil 12 drehbar und gegenüber dem Laufwagen 2 auch schwenkbar gelagert ist. Die nachfolgend näher erläuterte Verstellmechanik zwischen Laufwagen 2 und Drehring 3 ist durch einen Faltenbalg 13 abgedeckt.

Zur Verstellung des Drehringes 3 sind zwei Scherenarme 14, 15 vorgesehen, deren eine Enden an den mit 16, 17 bezeichneten Gelenkstellen am Lagerteil 12 und deren andere Enden an den mit 18 und 19 bezeichneten Gelenkstellen am Laufwagen 2 angelenkt sind. Zwischen den Gelenkstellen 16 und 18 einerseits und 17 und 19 andererseits sind elektromotorisch angetriebene Spindelantriebe 20, 21 vorgesehen, welche über eine nicht dargestellte Steuereinrichtung individuell angesteuert werden können. Mittig zwischen den beiden Gelenkstellen 16, 17 (Abstand a) enthält das Lagerteil 12 eine Schwenkachse 22, an der das eine Ende eines Teleskoparmes 23 angelenkt ist, dessen anderes Ende starr am Laufwagen 2 befestigt ist. In Verbindung mit der vorbeschriebenen Scherenarmkonstruktion ist es möglich, durch entsprechende Steuerung der beiden elektromotorischen Spindelantriebe 20, 21 den Drehring einerseits bei gleich schnellem Antrieb beider Spindelantriebe parallel zum Laufwagen 2 zu verstellen, andererseits bei nicht gleichmäßiger Verstellung der beiden Antriebe um die Schwenkachse 22 so zu verschwenken, daß der Mittelpunkt 25 des Drehringes 3 eine Querbewegung (von etwa ± 40 mm) mit einem Radius R von 350 mm in der mit 26 bezeichneten Pfeilrichtung ausführt. In Verbindung mit der Eigendrehbewegung, die der Drehring 3 auch noch um seine Mittelpunktachse 25 ausführen kann, wozu ein weiterer Antrieb vorgesehen ist, dessen Antriebs- und Führungsrollen mit 27 bezeichnet sind, kann so der Bewegungsablauf, welcher bei bekannten Geräten dieser Gattung mittels aufwendiger Mechanik erzielt wird, auf relativ einfache Weise nachvollzogen werden.

Die erwähnten Antriebs- und Führungsrollen 27 sind an Ober- und Unterseite des Drehringes 3 so angeordnet, daß sie eine Dreieckslagerung bilden. Zweckmäßigerweise ist an der Oberseite in der Mitte eine Antriebsrolle und beidseitig davon an der Unterseite je eine Führungsrolle vorgesehen. Das Lagerteil 12 ist hierzu entsprechend winkelig ausgebildet.

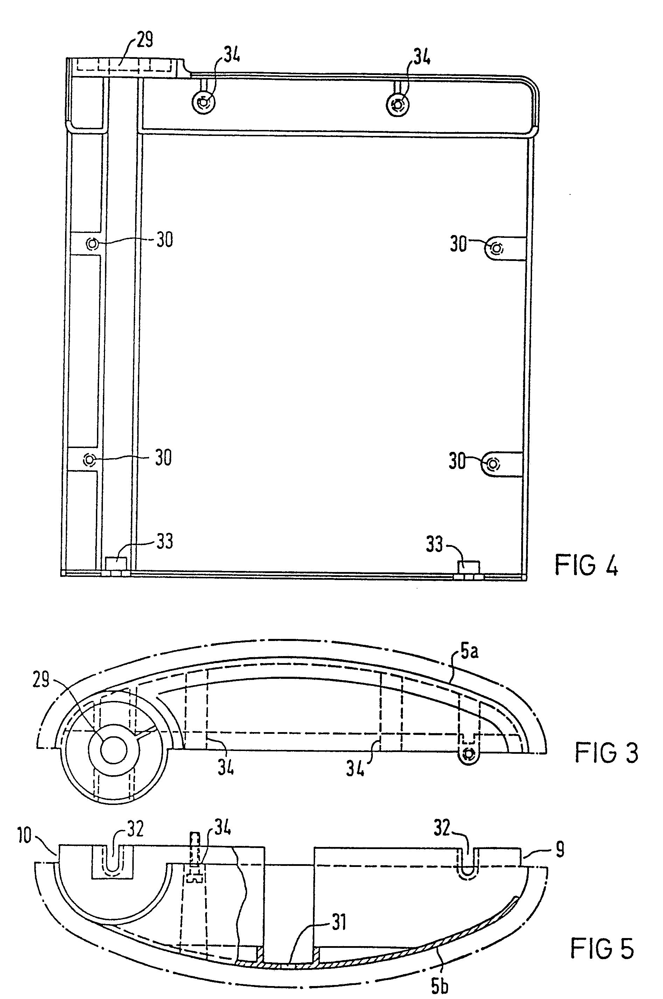

Der Filmkassettenhalter 5 besteht aus zwei durch eine in Figur 2 mit 28 bezeichnete Längsteilung gebildete Hälften 5a, 5b (Halbschalen), die in den Figuren 3 und 5 im Grundriß (Draufsicht) dargestellt sind.

Die Figuren 3 und 4 zeigen im Grund- und Aufriß die dem Strahler 4 abgewandte eine Gehäusehälfte 5a; diese enthält einerseits eine Lagerbuchse 29 zur Aufnahme des für die Anlenkung an den Tragarm 8 notwendigen Achslagers 7 und andererseits vier Befestigungseinrichtungen 30 für das in den Figuren 6 und 7 näher dargestellte Trägerteil, welches den in Figur 2 allgemein mit 40 bezeichneten Antrieb für die Filmkassette 11 aufnimmt.

Die in Figur 5 in der Draufsicht und teilweise im Schnitt dargestellte zweite Gehäusehälfte 5b enthält lediglich eine mit 31 bezeichnete Sekundärblende, die hier vorzugsweise Bestandteil des Filmkassettenhaltergehäuses selbst ist. Zu diesem Zweck besteht zumindest diese Gehäusehälfte 5b aus Zinkdruckguß, welches zur Absorption von Streustrahlung besonders gut geeignet ist. Zur Montage der Gehäusehälften 5a, 5b sind Längsschlitze 32 im einen Gehäuseteil 5b vorgesehen, welche in entsprechende Zapfen 33 des anderen Gehäuseteils 5a eingreifen. Darüber hinaus sind an beiden Gehäusehälften an den mit 34 bezeichneten Stellen Schraubverbindungen vorgesehen, die die beiden Gehäusehälften im montierten Zustand zusammenhalten. Hier ergeben sich dann auch an den beiden Stirnseiten der Gehäuseteile die bereits erwähnten schlitzförmigen Öffnungen 9, 10 zum Einführen sowie für den Auswurf der Filmkassette.

Die Figuren 6 und 7 zeigen einerseits in Draufsicht (Figur 6) und andererseits in Seitenansicht (Figur 7) einen in das Gehäuseteil 5a einsetzbaren Trägerteil 35 zur Aufnahme des kompletten Filmkassettenantriebs 40.

Dieser besteht aus einem mit 36 bezeichneten Elektromotor mit Getriebe, einer an der Antriebsachse befestigten Zwillingsrolle 37 und zwei in einem etwa der Filmkassettendicke entsprechenden Abstand dazu angeordneten Gegendruckrollen 38. Die Zwillingsrolle 37 ist mit einer Gummi- oder gummiähnlichen Auflage versehen oder besteht vollständig aus einem solchen Material. Die Gegendruckrollen bestehen aus Stahl. Der zwischen den Mantelflächen der Zwillingsrolle 37 und der Gegendruckrollen 38 gebildete Abstand ist so bemessen, daß die flexible Filmkassette durch Friktion transportiert werden kann. Sowohl die Zwillingsrollen als auch die beiden Gegendruckrollen sind durch geeignete Elemente, vorzugsweise Blattfedern 39, federelastisch aufgehängt. Der gesamte Träger 35 mit Antrieb 40 ist an den bereits erwähnten Stellen im Gehäuseteil 5a so befestigt, daß die Zwillingsrolle und die beiden Gegendruckrollen in der dargestellten Dreieckanordnung eng benachbart der Sekundärblende 31 angeordnet sind. Anstelle der Zwillingsrolle kann auch eine beispielsweise in gummielastischen Ringen gehalterte Walze vorgesehen sein.

## Patentansprüche

1. Zahnärztliche Röntgendiagnostikeinrichtung für Panorama-Schichtaufnahmen, mit einer Röntgenstrahlenquelle (4) und einem Halter (5) für eine Filmkassette (11), die an einem gemeinsamen Träger (3) gehaltert, einander gegenüberstehend angeordnet sind und mittels einer ersten Verstellvorrichtung (27) um das Objekt bewegt werden und mit einer Filmkassette (11), welche über eine am Halter (5) angeordnete, schlitzförmige Öffnung (9, 10) ein- und ausführbar ist und welche mittels einer zweiten Verstellvorrichtung (40) während der Aufnahme relativ zur Strahlenquelle (4) bewegbar ist wobei die Verstellvorrichtung für die Filmkassette einen im Gehäuse (5a, 5b) des Filmkassettenhalters (5) in Strahlungsrichtung gesehen hinter der Schlitzblende (31) angeordneten elektromotorischen Antrieb (40) vorsieht, der wenigstens ein Antriebsglied (37) und wenigstens ein korrespondierend angeordnetes Gegenglied (38) enthält, sowie mit wenigstens einer im Strahlengang zwischen Strahlenquelle und Filmkassette angeordneten Schlitzblende (31), *dadurch gekennzeichnet*, daß die den zu belichtenden Film einschließende Filmkassette (11) flexibel ist und daß die Verstellvorrichtung die flexible Filmkassette (11) mit Friktion entlang der Schlitzblende (31) vorbeibewegt, wobei mindestens eines der Glieder (37, 38), vorzugsweise das Antriebsglied (37), durch Eigenfederung und/oder mittels federnder Andruckelemente (39) gegen die flexible Filmkassette (11) gedrückt wird.

2. Röntgendiagnostikeinrichtung nach Anspruch 1, *dadurch gekennzeichnet*, daß als Antriebs- und Gegendruckglied Rollkörper (37, 38) vorgesehen sind.

3. Röntgendiagnostikeinrichtung nach Anspruch 2, *dadurch gekennzeichnet*, daß als Antriebsglied eine Zwillingsrolle (37) und als Gegenglied zwei unmittelbar benachbart der Schlitzblende (31) angeordnete, in Lagern frei drehbar geführte Gegendruckrollen (38) vorgesehen sind.

4. Röntgendiagnostikeinrichtung nach Anspruch 3, *dadurch gekennzeichnet*, daß zumindest die Mantelfläche der Zwillingsrollen (37) aus gummielastischem Material, die der Gegendruckrollen (38) dagegen aus Metall bestehen.

5. Röntgendiagnostikeinrichtung nach Anspruch 4, *dadurch gekennzeichnet*, daß die Gegendruckrollen (38) sich über die Höhe der Filmkassette (11) erstrecken.

6. Röntgendiagnostikeinrichtung nach einem der Ansprüche 1 bis 5, *dadurch gekennzeichnet*, daß zumindest das Antriebsglied (37) und dessen Antriebsmotor (36) an einem im Gehäuse (5a) des Filmkassettenhalters (5) lösbar angeordneten Träger (35) angeordnet ist.

7. Röntgendiagnostikeinrichtung nach Anspruch 1, *dadurch gekennzeichnet*, daß das Gehäuse des Filmkassettenhalters (5) aus zwei durch Längsteilung (28) gebildeten Hälften (5a, 5b) besteht, von denen die eine (5b) die Sekundärblende (31) und die andere (5a) die Antriebsteile (40) enthält.

8. Röntgendiagnostikeinrichtung nach einem der Ansprüche 1 bis 7, *dadurch gekennzeichnet*, daß zumindest die die Schlitzblende (31) beinhaltende Gehäusehälfte (5b) aus Zink-Druckguß besteht.

9. Röntgendiagnostikeinrichtung nach einem der Ansprüche 1 bis 8, *dadurch gekennzeichnet*, daß das Gehäuse des Filmkassettenhalters (5) um eine außermittig gelegene vertikale Achslagerung (29) schwenkbar am Träger (3) gehalten ist.

## Claims

1. Dental X-ray diagnosis device for panorama-layered-exposures, with an X-ray source (4) and a holder (5) for a film magazine (11) which are held on a common carrier (3) arranged opposite each other, and are moved around the object by means of a first adjusting device (27), and with a film magazine which is able to be led in and led out by way of a slot-shaped opening (9, 10) arranged on the holder (5), and which is able to be moved relative to the source of rays (4) during the exposure by means of a second adjusting device (40), whereby the adjusting device for the film magazine provides an electric motor drive (40) arranged in the housing (5a, 5b) of the film magazine holder (5) behind the slotted diaphragm (31) as seen in the direction of the rays, said drive containing at least one drive member (37) and at least one correspondingly arranged counter member (38), as well as with at least one slotted diaphragm (31), arranged in the path of rays, between the source of rays and the film magazine, characterised in that the film magazine (11), including the film to be exposed, is flexible and in that the adjusting device moves past the flexible film magazine (11) with friction along the slotted diaphragm (31), whereby at least one of the members (37, 38), preferably the drive member (37), is pressed by its elasticity and/or by means of elastic pressure elements (39) against the flexible film magazine (11).

2. X-ray diagnosis device according to claim 1, characterised in that rolling bodies (37, 38) are provided as the drive and counter pressure members.

3. X-ray diagnosis device according to claim 2, characterised in that provided as drive member there is a twin roller (37) and as counter member two counter pressure rollers (38) directly adjacent to the slotted diaphragm (31), guided in bearings in a freely rotatable manner.

4. X-ray diagnosis device according to claim 3, characterised in that at least the shell of the twin rollers (37) consists of rubber material and that of the counter pressure rollers (38) consists of metal.

5. X-ray diagnosis device according to claim 4, characterised in that the counter pressure rollers (38) extend over the height of the film magazine (11).

6. X-ray diagnosis device according to one of claims 1 to 5, characterised in that at least the drive member (37) and the drive motor (36) thereof is arranged on one carrier (35) arranged detachably in the housing (5a) of the film magazine holder (5).

7. X-ray diagnosis device according to claim 1, characterised in that the housing of the film magazine holder (5) consists of two halves (5a, 5b) formed by longitudinal partition (28), one (5b) of which contains the secondary diaphragm (31) and the other (5a) containing the drive parts (40).

8. X-ray diagnosis device according to one of claims 1 to 7, characterised in that at least the housing half (5b) containing the slotted diaphragm (31) consists of a zinc pressure die casting.

9. X-ray diagnosis device according to one of claims 1 to 8, characterised in that the housing of the film magazine holder (5) is held around an eccentrically laid vertical axle bearing (29) in a pivotable manner on the carrier (3).

## Revendications

1. Appareil de radiodiagnostic dentaire pour l'établissement de radiographies panoramiques, comportant une source (4) de rayons X et un élément (5) de retenue pour une cassette à film (11), qui sont disposés en vis-à-vis l'un de l'autre en étant maintenus sur un support commun (3) et sont déplacés autour de l'objet à l'aide d'un premier dispositif d'entraînement (27), et comportant une cassette à film (11), qui peut être introduite et ressortie à travers une ouverture en forme de fente (9, 10), ménagée dans l'élément de retenue (5) et peut être déplacée par rapport à la source de rayonnement (4), pendant la prise de la radiographie, au moyen d'un second dispositif d'entraînement (40), qui contient un système d'entraînement à moteur électrique disposé dans un logement (5a, 5b) de l'élément (5) de retenue de la cassette à film, en arrière du diaphragme à fente (31) lorsqu'on regarde dans la direction du rayonnement et comporte au moins un organe d'entraînement (37) et au moins un organe antagoniste (38) disposé de façon correspondante, et comportant au moins un diaphragme à fente (31) disposé dans le trajet du rayonnement entre la source de rayonnement et la cassette à film, caractérisé par le fait que la cassette à film (11), qui contient le film à exposer, est flexible et que le dispositif d'entraînement pour la cassette à film flexible (11) est déplacé avec friction le long du diaphragme à fente (31), auquel cas au moins l'un des organes (37, 38), de préférence l'organe d'entraînement (37), est repoussé par son élasticité propre et/ou au moyen d'éléments de poussée élastiques (39) contre la cassette à film flexible (11).

2. Appareil de radiodiagnostic suivant la revendication 1, caractérisé par le fait qu'on prévoit des corps roulants (37, 38) en tant qu'organe d'entraînement et organe de poussée antagoniste.

3. Appareil de radiodiagnostic suivant la revendication 2, caractérisé par le fait qu'il est prévu, comme organe d'entraînement, des galets jumelés (37) et, comme organe antagoniste, deux galets presseurs antagonistes (38) disposés directement au voisinage du diaphragme à fente (31) et guidés de manière à pouvoir tourner librement dans des paliers.

4. Appareil de radiodiagnostic suivant la revendication 3, caractérisé par le fait qu'au moins la surface enveloppe des galets jumelés (37) est réalisée en un matériau présentant l'élasticité du caoutchouc, tandis que les galets presseurs antagonistes (38) sont réalisés en un métal.

5. Appareil de radiodiagnostic suivant la revendication 4, caractérisé par le fait que les galets presseurs antagonistes (38) s'étendent sur la hauteur de la cassette à film (11).

6. Appareil de radiodiagnostic suivant l'une des revendications 1 à 5, caractérisé par le fait qu'au moins l'organe d'entraînement (37) et son moteur d'entraînement (36) sont disposés sur un support (35) monté de façon amovible dans le boîtier (5a) de l'élément (5) de retenue de la cassette à film.

7. Appareil de radiodiagnostic suivant la revendication 1, caractérisé par le fait que le boîtier du support (5) de la cassette à film est constitué par deux moitiés (5a, 5b), qui sont formées par une subdivision longitudinale (28) et dont l'une (5b) contient le diaphragme secondaire (31) alors que l'autre (5a) contient les éléments d'entraînement (40).

8. Appareil de radiodiagnostic suivant l'une des revendications 1 à 7, caractérisé par le fait qu'au moins la moitié (5b) du boîtier, qui contient le diaphragme à fente (31), est réalisée sous la forme d'une pièce en zinc, coulée sous pression.
9. Appareil de radiodiagnostic suivant l'une des revendications 1 à 8, caractérisé par le fait que le boîtier du support (5) de la cassette à film est maintenu dans l'élément (5) de retenue de la cassette à film autour d'un tourillon vertical décentré (29).

FIG 1

FIG 2

FIG 4

FIG 3

FIG 5

FIG 6

FIG 7